# EUROPEAN PATENT APPLICATION

(11) **EP 3 639 742 A1**
(43) Date of publication of application: **22.04.2020**
(21) Application number: 19203858.6
(22) Date of filing: 17.10.2019
(51) Int. Cl.: A61B 5/08, A61B 5/083, A61B 5/097

(54) **SYSTEMS AND METHODS FOR MEASUREMENT OF GAS CONCENTRATION DIFFERENCE BETWEEN INHALATION AND EXHALATION**

(30) Priority: 17.10.2018 US 201862746641 P
(71) Applicant: The Feinstein Institute for Medical Research, Manhasset, NY 11030 (US); Nihon Kohden Corporation, Shinjuku-ku Tokyo 161-8560 (JP)
(72) Inventor: BECKER, Lance, Great Neck, New York 11023 (US); LAMPE, Josh, Groton, Massachusetts 01450 (US); SHINOZAKI, Koichiro, Manhasset, New York 11030 (US); SAEKI, Kota, Port Washington, New York 11050 (US); WEISNER, Steve, Bedford, Massachusetts 01730 (US); YAMAMORI, Shinji, Kokubunji-shi, Tokyo, 185-0023 (JP)
(74) Representative: De Clercq & Partners

(57) **Abstract**

A system including a first sampling portion configured to sample inhalation gas made up of a first gaseous mixture, a second sampling portion configured to sample exhalation gas made up of a second gaseous mixture, a gas analyzer configured to measure gas concentrations, a switching valve that controls flow of the sampled inhalation gas and the sampled exhalation gas to the gas analyzer so as to alternately measure concentration of gaseous components within the first and second gaseous mixtures, a humidity control system that maintains humidity within the first and second gaseous mixtures to a predetermined humidity level, and a calculation section configured to calculate concentration differences of the gaseous components between the first and second gaseous mixtures.

## Description

### RELATED APPLICATION

This application claims priority to and the benefit of U.S. Provisional Patent Application No. 62/746,641, filed October 17, 2018, the contents of which are incorporated herein by reference in their entirety.

### TECHNICAL FIELD

The present invention is directed to measurement of gas concentrations, and in particular is directed to systems and methods for measuring and comparing concentration of gaseous components within different gaseous mixtures.

### BACKGROUND

A clinician might measure the concentration of a specific type of gas within different gaseous mixtures and use this information to determine how the concentrations vary between the gas mixtures. This measured comparison of gas concentration might be useful for patient diagnosis or a clinical study. However, obtaining an accurate measurement of gas concentration in biological samples is very challenging. Condition of the gas, such as temperature, pressure, humidity, and density, can be a cause of measurement errors. These errors might be compounded when attempting to measure the concentration difference between two different gas flows because the conditions of the gases might differ from one another.

Humidity has a major effect on accuracy of the concentration measurement because humidity not only changes gas concentration, but also affects gas density, heat conduction, and heat capacity, and therefore sensor accuracy. For example, during expiration, the body breathes out a gas in which the partial pressure of water vapor is 46 mmHg at 37 degrees centigrade at ambient pressure. If the gas temperature is cooled down to the ambient temperature (20 degrees centigrade), condensation occurs and the partial pressure of water vapor becomes 18 mmHg. Most gas analyzers capture the water drops caused by condensation using a water reservoir and measure the gas concentration under a condition in which the relative humidity is controlled to a range of 30-100 %.

A mechanical ventilator is typically connected to a gas supplying system. Therefore, the inspiration gas does not normally have humidity (0% relative humidity). This humidity difference between inspiration (0%) and expiration (30-100 %) is a significant obstacle in achieving an accurate measurement of the gas concentration differences between inspiration and expiration in a patient who is mechanically ventilated.

An accurate measurement of comparison between concentration of a gaseous component during exhalation and concentration of the gaseous component during inhalation can be of value in a number of ways. For example, such a measurement is useful in determining dosage of a gaseous substance delivered to a patient, such as, for example, anesthetics applied during surgery or inhalation gas (e.g., NO, H₂, and H₂S) used for the study of some emerging therapies. It is also possible that, if the concentration difference of oxygen between inspiration and expiration is measured accurately, O₂ consumption of the body can be estimated. O₂ consumption is used in multiple disciplines, such as nutrition support, sport medicine, and cardiopulmonary functional studies, because the O₂ consumption represents global metabolic alternations in the body. However, most conventional measurement devices fail to provide an adequate real-time measurement of O₂ consumption, which is mainly due to inaccurate measurement of the concentration difference between inhalation and exhalation.

### SUMMMARY OF THE INVENTION

An object of the present invention is to provide a system and method for accurate measurement of the gas concentration difference between inhalation and exhalation of a patient.

A system according to an exemplary embodiment of the present invention comprises: a first sampling portion configured to sample inhalation gas made up of a first gaseous mixture; a second sampling portion configured to sample exhalation gas made up of a second gaseous mixture; a gas analyzer configured to measure gas concentrations; a switching valve that controls flow of the sampled inhalation gas and the sampled exhalation gas to the gas analyzer so as to alternately measure concentration of gaseous components within the first and second gaseous mixtures; a humidity control system that maintains humidity within the first and second gaseous mixtures to a predetermined humidity level; and a calculation section configured to calculate concentration differences of the gaseous components between the first and second gaseous mixtures.

In an exemplary embodiment, the first gas sampling portion is connected to an inhalation gas circuit of a mechanical ventilator, and the second gas sampling portion is connected to an exhaust port or an exhalation gas circuit of the mechanical ventilator.

In an exemplary embodiment, the first gas sampling portion is connected to an inhalation gas circuit of a breathing mask with a first one-way valve, and the second gas sampling portion is connected to an exhalation gas circuit of the breathing mask with a second one-way valve.

In an exemplary embodiment, the humidity control system comprises at least one of a membrane dryer unit, a refrigerating dryer unit or a desiccant.

In an exemplary embodiment, the humidity control system is a membrane dryer unit, and purge gas used for the membrane dryer unit is at least one of dry gas from a gas supplying system, dry gas from a gas tank or dry gas as dried by a desiccant.

In an exemplary embodiment, the humidity control system is a refrigerating unit, and the refrigerating unit lower temperature of the first and second gaseous mixtures to -20°C or below.

In an exemplary embodiment, the gas analyzer measures at least oxygen concentration and carbon dioxide concentration, and the first calculation section is configured to calculate oxygen concentration differences and carbon dioxide concentration differences.

In an exemplary embodiment, the calculation section is further configured to calculate respiratory quotient (RQ) based on the calculated concentration differences.

In an exemplary embodiment, the system further comprises a flow sensor that measures flow rate of at least one of the inhalation gas or the exhalation gas, wherein the calculation section is further configured to calculate oxygen consumption (VO₂) and carbon dioxide generation (VCO₂) based on the measured concentration differences and the flow rate.

In an exemplary embodiment, the flow sensor comprises a thermal sensor, a hygrometer and a pressure sensor.

In an exemplary embodiment, the first gas sampling portion comprises a first gas mixing chamber and the second gas sampling portion comprises a second gas mixing chamber.

A method for measuring biological information according to an exemplary embodiment of the present invention comprises: sampling inhalation gas made up of a first gaseous mixture; sampling exhalation gas made up of a second gaseous mixture; controlling flow of the sampled inhalation gas and the sampled exhalation gas to a gas analyzer so as to alternately measure concentration of gaseous components within the first and second gaseous mixtures; maintaining humidity within the first and second gaseous mixtures to a predetermined humidity level prior to measurement of the concentration of the gaseous components; and calculating concentration differences of the gaseous components between the first and second gaseous mixtures.

In an exemplary embodiment, the method further comprises the steps of: connecting the first gas sampling portion to an inhalation gas circuit of a mechanical ventilator; and connecting the second gas sampling portion to an exhaust port or an exhalation gas circuit of the mechanical ventilator.

In an exemplary embodiment, the method further comprises the steps of: connecting the first gas sampling portion to an inhalation gas circuit of a breathing mask with a first one-way valve; and connecting the second gas sampling portion to an exhalation gas circuit of the breathing mask with a second one-way valve.

In an exemplary embodiment, the humidity is maintained by a humidity control system comprising at least one of a membrane dryer unit, a refrigerating dryer unit or a desiccant.

In an exemplary embodiment, the humidity control system is a membrane dryer unit, and purge gas used for the membrane dryer unit is at least one of dry gas from a gas supplying system, dry gas from a gas tank or dry gas as dried by a desiccant.

In an exemplary embodiment, the humidity control system is a refrigerating unit, and the refrigerating unit lower temperature of the first and second gaseous mixtures to -20°C or below.

In an exemplary embodiment, the gas analyzer measures at least oxygen concentration and carbon dioxide concentration, and the method further comprises the step of calculating oxygen concentration differences and carbon dioxide concentration differences.

In an exemplary embodiment, the method further comprises the step of calculating respiratory quotient (RQ) based on the calculated concentration differences.

In an exemplary embodiment, the method further comprises the steps of: measuring flow rate of at least one of the inhalation gas or the exhalation gas; and calculating oxygen consumption (VO₂) and carbon dioxide generation (VCO₂) based on the measured concentration differences and the flow rate.

These and other features and advantages of the present invention will be presented in more detail in the following detailed description and the accompanying figures which illustrate by way of example principles of the invention.

### DESCRIPTION OF THE DRAWINGS

The features and advantages of exemplary embodiments of the present invention will be more fully understood with reference to the following, detailed description when taken in conjunction with the accompanying figures, wherein:
FIG. 1 is a block diagram of a conventional system for measuring gas concentration differences between gaseous mixtures;
FIG. 2 is a block diagram of a system for measuring biological function according to an exemplary embodiment of the present invention;
FIG. 3 is a block diagram of a membrane dryer unit according to an exemplary embodiment of the present invention;
FIG. 4 is a block diagram of a refrigerating dryer unit according to an exemplary embodiment of the present invention;
FIG. 5 is a block diagram of a system for measuring biological function according to another exemplary embodiment of the present invention; and
FIG. 6 is block diagram of a system for measuring biological function according to another exemplary embodiment of the present invention;

### DETAILED DESCRIPTION

A gas concentration measurement system according to an exemplary embodiment of the present invention controls the humidity of gases in real-time to a predetermined humidity level (*e.g.,* 1 mmHg water pressure, 2 mmHg water pressure, 3 mmHg water pressure, 20 mmHg water pressure) before the gases from different sources, or streams, are alternately passed through to a sensor for measurement of the gas component concentrations. Calculation of the difference between gas concentration values obtained by one sensor decreases the error propagation that would otherwise occur with multiple sensors. This allows for a more accurate gas concentration measurement as compared to any other currently available gas concentration measurement system.

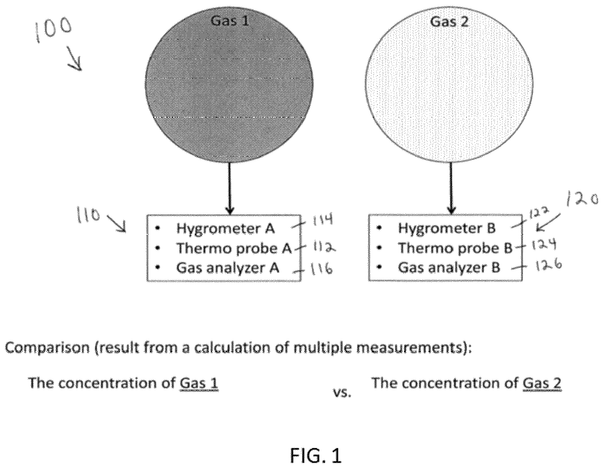

FIG. 1 is a block diagram of a system, generally designated by reference number 100, for measuring and calculating the difference in concentration of a gas between two different gaseous mixtures (*e.g.,* O₂ concentration difference between inspired and expired air). The system 100 measures the concentration of a gas within two different gas streams, Gas 1 and Gas 2, and thus includes two sets of sensors. Specifically, the system 100 includes a first sensor set 110 used to obtain relevant data for Gas 1 and a second sensor set 120 used to obtain relevant data for Gas 2. First sensor set 110 includes first thermometer 112, first hygrometer 114 and first gas analyzer 116, and second sensor set 120 includes second thermometer 122, second hygrometer 124 and second gas analyzer 126. The system 100 has diminished accuracy because the sensors within each set are used independently, which causes propagation of errors.

In order to reduce the errors associated with the duplicate sensor systems described above, a system for measuring gas concentration difference in accordance with an exemplary embodiment of the present invention includes the use of only one gas analyzer so that the gas concentrations within different gaseous mixtures can be measured under the same humidity conditions.

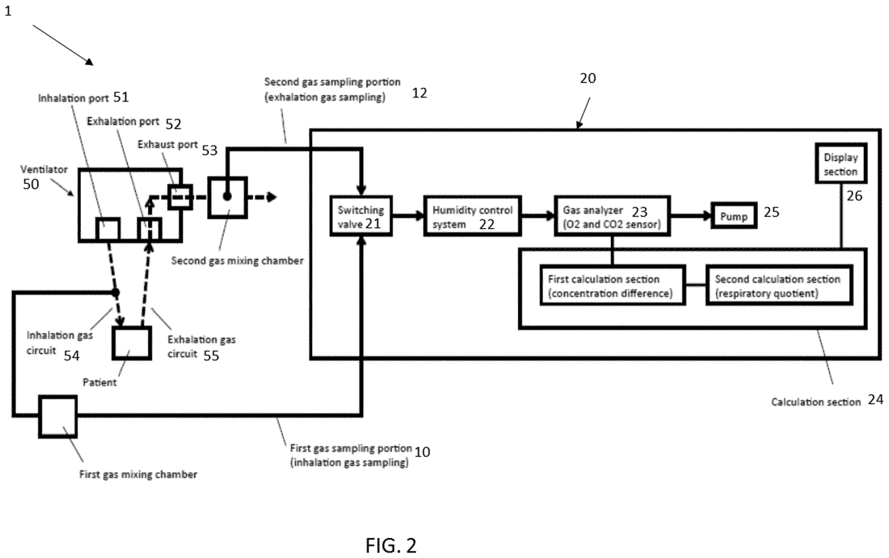

FIG. 2 is a block diagram of a system, generally designated by reference number 1, for measuring and calculating the difference in concentration of a gas between two different gaseous mixtures according to an exemplary embodiment of the present invention. In the present embodiment, the system 1 is used with a patient mechanically ventilated by a ventilator 50. The ventilator 50 includes an inhalation port 51, an exhalation port 52, an exhaust port 53, an inhalation gas circuit 54 and an exhalation gas circuit 55. The system 1 includes a first gas sampling portion 10 connected to the inhalation gas circuit 54 of the ventilator 50 and a second gas sampling portion 12 connected to either the exhalation gas circuit 55 or the exhaust port of the ventilator 50. The first gas sampling portion 10 may be intended for sampling of the inhalation gas of the ventilated patient and the second sampling portion 12 may be intended for sampling of the exhalation gas of the ventilated patient. The system 1 further includes a gas concentration measurement module 20 that includes a switching valve 21, a humidity control system 22, a gas analyzer 23, a calculation section 24, a pump 25 and a display section 26. The switching valve 21 alternately directs the inhalation gas and the exhalation gas to the gas concentration measurement module 20. In exemplary embodiments, the switching valve 21 might be a solenoid valve, a pneumatic valve, or a mechanical valve (manual or motorized).

The humidity control system 22 may include components that increase and/or decrease the humidity of the gas flowing through the gas concentration measurement module 20 based on feedback measurements to maintain the humidity at a specific level or within a predetermined range. For example, the humidity control system 22 may include a membrane dryer unit, a refrigerating dryer unit or a desiccant.

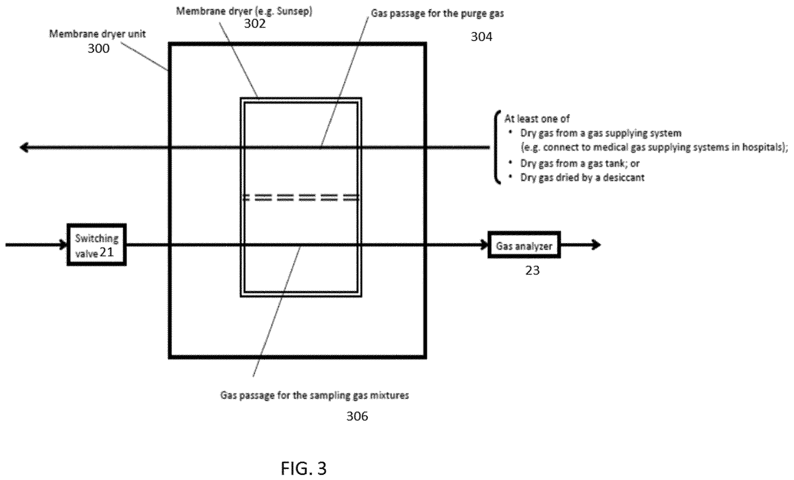

FIG. 3 is a block diagram of a membrane dryer unit, generally designated by reference number 300, according to an exemplary embodiment of the present invention. As known in the art, the membrane dryer unit 300 includes a membrane dryer 302, a dry gas passage 304 and a sampling gas passage 306. Suitable membrane dryer units for use in exemplary embodiments of the present invention include the Sunsep™ Membrane Type Gas Dryer (AGC Engineering, Japan) and Gas Sample Drying Products with Nafion™ tubing (Perma Pure LLC, Lakewood, NJ, USA). In embodiments, the dry gas (or purge gas) may be sourced from a gas supplying system, a gas tank and/or a gas previously dried by a desiccant.

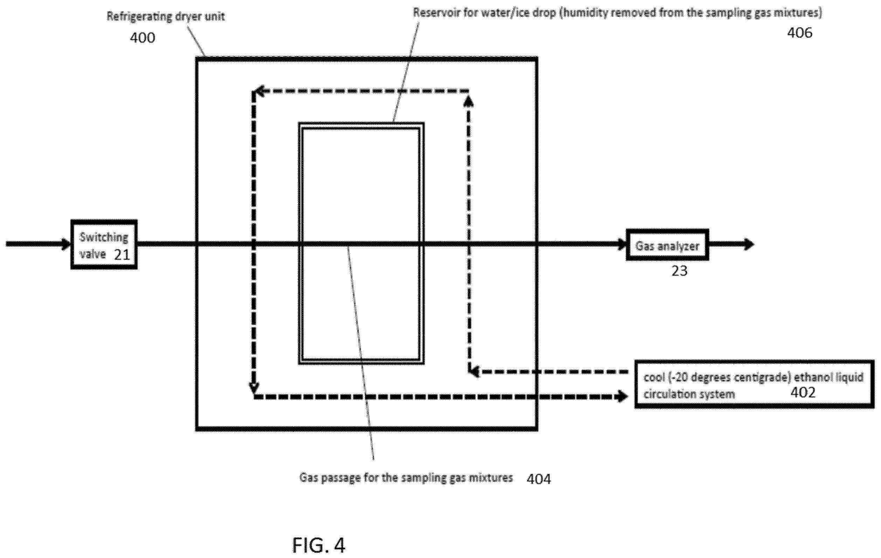

FIG. 4 is a block diagram of a refrigerating dryer unit 400 according to an exemplary embodiment of the present invention. As known in the art, the refrigerating unit 400 includes a cold liquid circulation system 402, a sampling gas passage 404 and a reservoir 406. Suitable refrigerating dryer units for use in exemplary embodiments of the present invention include F25-ME Heating/Refrigerated Circulator (JULABO USA, Inc., Allentown, PA, USA) and MX07R-20 Refrigerating/Heating Bath (PolyScience, Niles, Illinois, USA). In embodiments, the refrigerating dryer unit 400 may decrease temperature of the sampling gas to equal to or below -20°C. A cooled gas at a temperature of 5 °C holds a capacity of the partial pressure of water gas at 6.9 mmHg at atmospheric pressure (relative humidity of 15-40 %). By comparison, a gas at a temperature of -20 °C holds the partial pressure of water at 1.1 mmHg (relative humidity of 2-6 %). Some oxygen sensors are very sensitive to the humidity because changes in humidity cause differences in heat conductance and capacity, which in turn effects the sensor accuracy. Thus, the humidity of the gas flowing through the sensors is preferably held at a very low range.

As mentioned, a desiccant may also be used to dry the sampling gas flowing into the gas concentration measurement module 20. Examples of suitable desiccants include montmorillonite clay, silica gel, molecular sieve, calcium oxide and calcium sulfate.

The gas analyzer 23 is configured to measure at least the oxygen concentration and the carbon dioxide concentration in the inhalation and exhalation gases. The gas analyzer 23 might be, for example, an infrared gas analyzer, and more specifically, in the case of O₂ sensing, might be a paramagnetic sensor, electro-galvanic oxygen sensor, zirconia sensor, fiber-optic sensor based on fluorescence quenching, or use laser absorption spectroscopy, and in the case of CO₂ sensing, might be a chemical sensor, or use nondispersive infrared spectroscopy (NDIR) or Fourier-transform infrared spectroscopy (FTIR).

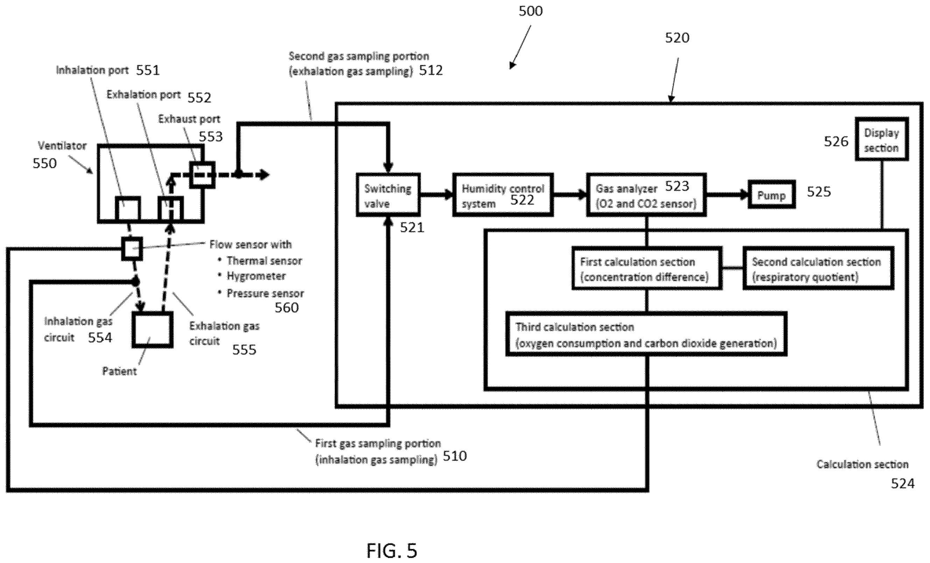

The calculation section 24 may be a computer hardware component that includes a memory unit and a processor, where the memory has stored thereon processor readable code that, when read by the processor, carries out operations to calculate one or more of the following parameters: oxygen concentration difference (between inhalation and exhalation), carbon dioxide concentration difference (between inhalation and exhalation), respiratory quotient (RQ), oxygen consumption (VO₂) and carbon dioxide generation (VCO₂). In this regard, respiratory quotient (RQ) may be calculated based on the measured CO₂ and O₂ concentration differences, and oxygen consumption (VO₂) and carbon dioxide generation (VCO₂) may be calculated based on the concentration differences and flow rate of at least one of the inhalation gas or the exhalation gas. In order to calculate oxygen consumption (VO₂) and carbon dioxide generation (VCO₂), in accordance with another exemplary embodiment illustrated in FIG. 5, the system (generally designated by reference number 500 in FIG. 5) may further include a flow sensor 560 that measures the flow rate of the gas. The flow sensor 560 may includes a thermal sensor that measures gas temperature, a hygrometer that measures gas humidity and a pressure sensor that measures gas pressure. As in the previously-described embodiment, the system 500 may include a ventilator 550 with an inhalation port 551, an exhalation port 552, an exhaust port 553, an inhalation gas circuit 554 and an exhalation gas circuit 555. The system 500 includes a first gas sampling portion 510 connected to the inhalation gas circuit 554 of the ventilator 550 and a second gas sampling portion 512 connected to either the exhalation gas circuit 555 or the exhaust port of the ventilator 550. The first gas sampling portion 510 may be intended for sampling of the inhalation gas of the ventilated patient and the second sampling portion 512 may be intended for sampling of the exhalation gas of the ventilated patient. The system 500 further includes a gas concentration measurement module 520 that includes a switching valve 521, a humidity control system 522, a gas analyzer 523, a calculation section 524, a pump 525 and a display section 526. The switching valve 521 alternately directs the inhalation gas and the exhalation gas to the gas concentration measurement module 520. In exemplary embodiments, the switching valve 521 might be a solenoid valve, a pneumatic valve, or a mechanical valve (manual or motorized).

Also, as shown in FIG. 2, the system 1 (and although not shown, the system 500) may include a mixing chamber within the first gas sampling portion 10 for collection of the inhalation gas and another mixing chamber within the second gas sampling portion 12 for collection of the exhalation gas.

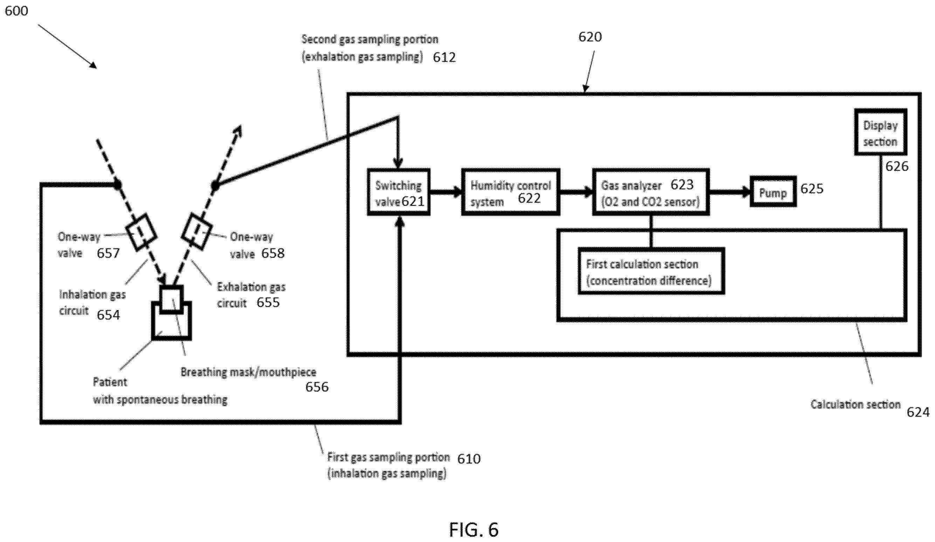

FIG. 6 is a block diagram of a system, generally designated by reference number 600, for measuring and calculating the difference in concentration of a gas between two different gaseous mixtures according to another exemplary embodiment of the present invention. The system 600 may include components similar to those of the system 1, except that the system 600 is configured for operation with a spontaneously breathing patient (*i.e.,* the patient is not breathing with the aid of a ventilator). Rather than a ventilator, the patient may be fitted with a breathing mask 656 that includes an inhalation gas circuit 654 and an exhalation gas circuit 655. The first gas sampling portion 610 may be connected to the inhalation gas circuit 654 with a first one-way valve 657 and the second gas sampling portion 612 may be connected to the exhalation gas circuit 655 with a second one-way valve 658 to ensure that inhalation gas and exhalation gas are kept separate from one another. The system 600 further includes a gas measurement module 620 that includes a switching valve 621, a humidity control system 622, a gas analyzer 623, a calculation section 624, a pump 625 and a display section 626. The switching valve 621 alternately directs the inhalation gas and the exhalation gas to the gas concentration measurement module 620. As described with reference to the previous embodiment, the gas measurement module 620 may be configured to calculate gas concentration differences, RQ, VCO₂, VO₂, as well as dosages of gaseous substance delivered to a patient, such as anesthesia gas, NO, H₂, H₂S etc.

The following Example and Comparative Example illustrates advantages of the present invention.

### COMPARATIVE EXAMPLE

A Douglas Bag method was used to measure concentration of O₂ and CO₂ in inhaled and exhaled air of a test lung (QuickLung Breather, IngMar Medical, Pittsburgh, USA) operated by a mechanical ventilator (AVEA ventilation system, CareFusion, Yorba Linda, CA, USA). A 50 L PVC bag (Douglas Bag, Harvard Apparatus, Holliston, MA, USA) and a 3.8 L polyvinylidene fluoride (PVDF) bag (Dual-Valve Kynar PVDF Bag, Cole-Parmer Kynar, Vernon Hills, IL, USA) were prepared. The exhaled and inhaled gases were continuously collected into the 50 L PVC bag and the 3.8 L PVDF bag, respectively. The 50 L PVC bag was connected to the exhaust port of the mechanical ventilator so that all the exhaled gas was collected in the bag. A part of the inhaled gas (10-15 % of the ventilation volume) was collected into the 3.8 L PVDF bag from an adapter port that was added into the ventilator circuit. The exhaled and inhaled gases were collected simultaneously and a total gas collection time was 7-8 minutes for each experiment. After the gas collection was completed, the two sealed bags were frozen inside a freezer (FUM 21SVCRWW, General Electric, Louisville, KY, USA). The temperatures of the bag surface and the sample gas were monitored and the gas concentration measurements were started after the temperatures reached below -20 degrees centigrade. Two sampling tubes were prepared and disposed in the freezer for each bag, and a gas analyzer, which was setup outside the freezer, obtained the exhaled and inhaled gas separately through the tubes from outside. A 3-way stop connector was connected to the two sampling tubes and the gas analyzer so that the analyzer always obtained one of the gas samples, with the input to the gas analyzer switched by changing the gas flow direction of the 3-way stop connector. The gas analyzer used to measure the gas concentrations was a GF-210R model, produced by Nihon Kohden Corporation of Tokyo, Japan.

### EXAMPLE

The same test lung, mechanical ventilator and gas analyzer from the Comparative Example were used to measure CO₂ and O₂ concentrations in inhaled and exhaled air, but instead of using the Douglas Bag method, the test was performed using a system including two chambers (one for each of inhaled gas and exhaled gas), a humidity control system for equilibrating humidity levels within each chamber, a single gas analyzer and a switching valve to control gas flow from the chambers to the gas analyzer. This was a conceptual proof experiment of a real-time measurement with humidity control. Two separate 250 mL glass bins were prepared. These glass bins were frozen inside a dry ice container. The exhaled gas and the inhaled gas were sampled directly from the mechanical ventilation circuit through a sampling port added into the ventilation circuit. The exhaled (exhaust) gas was passed through one of the bins and the inhaled gas was passed through another bin. The gas analyzer was connected downstream of the gas flows so that the gas analyzer obtained a gas after the humidity was controlled by the icy bins. Gas sampling rate was controlled by the gas analyzer at 200 mL/min and a gas flow direction was switched by a 3-way stop connector so that the analyzer obtained one of the gas samples. The inhaled gas was humidified by adding a water reservoir into the mechanical ventilation circuit. In the experimental setting, the humidity level measured at the inhalation port was < 0% (under detection limit) and the humidity level at the exhaust port was 40-70 %. The humidity in both gases became the same level of < 0% after the gas samples were passed through each icy bin.

Table 1 below shows the results of the Comparative Example and the Example (three Trials were performed in each example), where FIO₂ indicates the O₂ concentration (%) in the inhalation gas; FEO₂ indicates the O₂ concentration (%) in the exhalation gas; FECO₂ indicates the CO₂ concentration (%) in the exhalation gas; and RQ is calculated by FECO₂/(FIO₂-FEO₂).

**TABLE 1**

| Trial | Example | FIO₂ | FEO₂ | FIO₂-FEO₂ | FECO₂ | RQ |
|---|---|---|---|---|---|---|
| 1 | EXAMPLE | 89.51 | 86.88 | 2.63 | 2.51 | 0.95 |
| | COMPARATIVE EXAMPLE | 89.29 | 86.69 | 2.60 | 2.52 | 0.97 |
| 2 | EXAMPLE | 88.24 | 85.58 | 2.66 | 2.52 | 0.94 |
| | COMPARATIVE EXAMPLE | 88.14 | 85.33 | 2.81 | 2.53 | 0.90 |
| 3 | EXAMPLE | 88.19 | 84.89 | 3.30 | 2.64 | 0.80 |
| | COMPARATIVE EXAMPLE | 88.09 | 84.90 | 3.18 | 2.56 | 0.81 |

The data in Table 1 shows that the results of Example 1 are close to the values obtained using the Douglas Bag method in the Comparative Example, and therefore may be considered accurate results. This demonstrates that the real-time measurements of gas samples directly collected from the ventilator circuits have equal values with the measurements in a steady state, where the gas concentration was measured by the traditional Douglas Bag method.

Now that embodiments of the present invention have been shown and described in detail, various modifications and improvements thereon will become readily apparent to those skilled in the art. Accordingly, the spirit and scope of the present invention is to be construed broadly and not limited by the foregoing specification.

## Claims

1. A system comprising:
a first sampling portion configured to sample inhalation gas made up of a first gaseous mixture;
a second sampling portion configured to sample exhalation gas made up of a second gaseous mixture;
a gas analyzer configured to measure gas concentrations;
a switching valve that controls flow of the sampled inhalation gas and the sampled exhalation gas to the gas analyzer so as to alternately measure concentration of gaseous components within the first and second gaseous mixtures;
a humidity control system that maintains humidity within the first and second gaseous mixtures to a predetermined humidity level; and
a calculation section configured to calculate concentration differences of the gaseous components between the first and second gaseous mixtures.

2. The system of claim 1, wherein the first gas sampling portion is connected to an inhalation gas circuit of a mechanical ventilator, and the second gas sampling portion is connected to an exhaust port or an exhalation gas circuit of the mechanical ventilator.

3. The system of claim 1, wherein the first gas sampling portion is connected to an inhalation gas circuit of a breathing mask with a first one-way valve, and the second gas sampling portion is connected to an exhalation gas circuit of the breathing mask with a second one-way valve.

4. The system of any one of claims 1 to 3, wherein the humidity control system comprises at least one of a membrane dryer unit, a refrigerating dryer unit or a desiccant, wherein when the humidity control system is a membrane dryer unit, optionally purge gas used for the membrane dryer unit is at least one of dry gas from a gas supplying system, dry gas from a gas tank or dry gas as dried by a desiccant.

5. The system of claim 4, wherein the humidity control system is a refrigerating unit, and the refrigerating unit lower temperature of the first and second gaseous mixtures to - 20°C or below.

6. The system of any one of claims 1 to 5, wherein the gas analyzer measures at least oxygen concentration and carbon dioxide concentration, and the first calculation section is configured to calculate oxygen concentration differences and carbon dioxide concentration differences and is optionally further configured to calculate respiratory quotient (RQ) based on the calculated concentration differences.

7. The system of claim 6, further comprising a flow sensor that measures flow rate of at least one of the inhalation gas or the exhalation gas, wherein the calculation section is further configured to calculate oxygen consumption (VO₂) and carbon dioxide generation (VCO₂) based on the measured concentration differences and the flow rate, preferably wherein the flow sensor comprises a thermal sensor, a hygrometer and a pressure sensor.

8. The system of any one of claims 1 to 7, wherein the first gas sampling portion comprises a first gas mixing chamber and the second gas sampling portion comprises a second gas mixing chamber.

9. A method for measuring biological information, comprising:
sampling inhalation gas made up of a first gaseous mixture;
sampling exhalation gas made up of a second gaseous mixture;
controlling flow of the sampled inhalation gas and the sampled exhalation gas to a gas analyzer so as to alternately measure concentration of gaseous components within the first and second gaseous mixtures;
maintaining humidity within the first and second gaseous mixtures to a predetermined humidity level prior to measurement of the concentration of the gaseous components; and
calculating concentration differences of the gaseous components between the first and second gaseous mixtures.

10. The method of claim 9, further comprising the steps of:
connecting the first gas sampling portion to an inhalation gas circuit of a mechanical ventilator; and
connecting the second gas sampling portion to an exhaust port or an exhalation gas circuit of the mechanical ventilator.

11. The method of claim 9, further comprising the steps of:
connecting the first gas sampling portion to an inhalation gas circuit of a breathing mask with a first one-way valve; and
connecting the second gas sampling portion to an exhalation gas circuit of the breathing mask with a second one-way valve.

12. The method of any one of claims 9 to 11, wherein the humidity is maintained by a humidity control system comprising at least one of a membrane dryer unit, a refrigerating dryer unit or a desiccant, wherein when the humidity control system is a membrane dryer unit, optionally purge gas used for the membrane dryer unit is at least one of dry gas from a gas supplying system, dry gas from a gas tank or dry gas as dried by a desiccant and wherein when the humidity control system is a refrigerating unit, optionally the refrigerating unit lower temperature of the first and second gaseous mixtures to -20°C or below.

13. The method of any one of claims 9 to 12, wherein the gas analyzer measures at least oxygen concentration and carbon dioxide concentration, and the method further comprises the step of calculating oxygen concentration differences and carbon dioxide concentration differences.

14. The method of claim 13, further comprising the step of calculating respiratory quotient (RQ) based on the calculated concentration differences.

15. The method of claim 13 or 14, further comprising the steps of:
measuring flow rate of at least one of the inhalation gas or the exhalation gas; and
calculating oxygen consumption (VO₂) and carbon dioxide generation (VCO₂) based on the measured concentration differences and the flow rate.
